# EUROPEAN PATENT SPECIFICATION

(11) **EP 0 723 954 B1**
(45) Date of publication and mention of the grant of the patent: **19.05.1999**
(21) Application number: 95300458.7
(22) Date of filing: 25.01.1995
(51) Int. Cl.: C07C 253/34

(54) **Acetonitrile purification via an adsorption-based process**
Verfahren zur Reinigung von Acetonitril durch Adsorptionsverfahren
Purification de l'acétonitrile via un procédé d'adsorption

(43) Date of publication of application: 31.07.1996
(73) Proprietor: THE STANDARD OIL COMPANY, Cleveland, Ohio 44114-2375 (US)
(72) Inventor: Attig, Thomas G., Aurora, Ohio 44202 (US); Cesa, Mark C., South Euclid, Ohio 44121 (US); Blachman, Marc W., Lyndhurst, Ohio 44124 (US)
(74) Representative: Perkins, Nicholas David

(56) References cited:
- WO-A-93/23366
- BE-A- 1 002 441
- US-A- 4 287 134
- DATABASE WPI Week 7834, Derwent Publications Ltd., London, GB; AN 78-61375A & JP-A-53 082 722 (NITTO CHEM IND) 21 July 1978
- DATABASE WPI Week 8806, Derwent Publications Ltd., London, GB; AN 88-041181 & SU-A-1 318 588 (CHEM REAGENT PURE) 23 June 1987

## Description

This invention relates to acetonitrile purification via an adsorption-based process. More particularly, this invention relates to a process for acetonitrile purification involving pretreating the acetonitrile to selectively convert unsaturated nitriles contained in the acetonitrile into products that can be preferentially eliminated. The pretreatment is followed by adsorption to remove the resulting products and other organic impurities present in the acetonitrile.

The acetonitrile purification process of the present invention can be used to up-grade acetonitrile presently produced by chemical plants to a higher purity material of signigicantly greater value. Specifically, removal of UV-absorbing organic impurities from acetonitrile results in a product with unusually high transparency in the UV and removal of water makes acetonitrile useful in certain commercial applications. Commercial acetonitrile typically contains water as well as trace amounts of acrylonitrile, crotonitrile, acetamide, and allyl alcohol. Applications for high-purity acetonitrile require a UV cutoff of less than 190nm as well as extremely dry material ([H₂0]<1ppm). The presence of organic impurities containing C=C and C=O functionalities, which absorb light in the 190-230nm region, results in the acetonitrile having a UV cutoff above 200 nm. This renders the acetonitrile material unacceptable, therefore requiring further processing by the producers of high-purity acetonitrile. The traditional commercial method of acetonitrile purification utilises a costly multi-step process involving permanganate oxidation, acid treating, phosphorous pentoxide drying, and two distillations.

Classical methods for purification of acetonitrile are summarised in D.D.Perrin, W.L.F.Armarego, and D.R.Perrin, Purification of Laboratory Chemicals, 2^{nd} Ed., Pergammon Press, New York, 1980, pp. 79-81. Water is removed by treating acetonitrile with silica gel, 4A molecular sieves, calcium hydride, or phosphoric anhydride, and distilling. Acetic acid and other carboxylic acids can be removed with alumina followed by distillation. Unsaturated nitriles can be removed by initial refluxing with a small amount of aqueous potassium hydroxide solution. However, this reference discloses that preliminary treatment of acetonitrile with cold, saturated aqueous potassium hydroxide is undesirable because of base-catalysed hydrolysis and the introduction of water. Unsaturated impurities present in the acetonitrile can be removed by treating the acetonitrile with alkaline potassium permanganate solutions but then further treatment to remove water is necessary.

In L.Carlsen, H.Egsgaard, J.J.Anderson, Analyt. Chem., 1979, 51(9), 1593-5, an acetonitrile purification process of treatment with strong base, flash distillation, treatment with a strong acid, fractional distillation, and drying with alumina is disclosed. In A.Hofmanova, K.Angelis, Chem. Listy, 1978, 72(3), 306-9, (CA 88: 179367s), acetonitrile is purified by boiling for 5 hours with 10% aqueous potassium hydroxide solution, adsorbing on aluminium oxide to remove carboxylic acid salts, drying with phosphoric anhydride or molecular sieve, then fractional distillation from phosphoric anhydride. These methods require multiple steps and involve the use of aqueous base solutions and subsequent distillations and other treatment to remove water and to achieve satisfactory purity.

H. Yu, Z. Xu, in Shengwu Huaxue Yu Shengwu Wuli Jinzhan, 1984, 55, 70-2 (CA 100: 127264c) describe treatment of acetonitrile with potassium permanganate-sodium hydroxide in methanol, followed by adsorption by passing through activated charcoal and acidified alumina. In Russia Patent SU 1,318,588, acetonitrile is passed through potassium permanganate adsorbed on alumina to reduce allyl alcohol concentrations to 4-6ppm and acrylonitrile to 1-3ppm. These references which disclose the use of potassium permanganate either do not disclose the removal of water after potassium permanganate treatment or involve cumbersome distillations, or require co-treatment with potassium permanganate and a strong base.

The main obstacle to effectively using adsorption for acetonitrile upgrading is the similarity in physical properties between acetonitrile and the unsaturated nitriles contained in the acetonitrile. In order for adsorption to be a viable approach to purification, the unsaturated nitriles must first be converted to other species having sufficiently different physical characteristics.

It is desirable to find a method for acetonitrile purification which does not utilise a costly and cumbersome multi-step process involving distillation.

It is an object of the present invention to provide a method for acetonitrile purification utilising adsorption-based technology to produce high-purity acetonitrile.

It is a further object to convert difficult-to-remove impurities such as unsaturated nitriles into more easily removed products such as oxygenates, aromatics, and polymers.

It is another object of this invention to provide an acetonitrile purification process using low cost processing equipment and materials, and having an overall processing cost significantly less than that incurred using traditional distillation-based technology.

It is still another object of the present invention to provide a method for acetonitrile purification which allows for flexibility in process design.

These and other objects, shall become apparent from the specification which follows and accomplished by the invention as hereinafter described and claimed.

To achieve the foregoing objects in accordance with the purpose of the invention as embodied and broadly described herein, there is provided a method for acetonitrile purification using an adsorption-based process. The invention is directed to a process for purifying acetonitrile containing impurities comprising unsaturated nitriles and other materials with C=C and C=O functional groups. The process is comprised of the steps of pretreating the acetonitrile by adding a solid reagent to selectively convert the unsaturated nitriles contained in the acetonitrile into products having different physical characteristics than acetonitrile and passing the acetonitrile through a series of adsorbent beds to remove the resulting products and other impurities in the acetonitrile.

This invention is directed to a method for acetonitrile purification employing an adsorption-based process. The acetonitrile purification process comprises pretreating the acetonitrile containing organic impurities, such as unsaturated nitriles, by adding a solid reagent to selectively convert the unsaturated nitriles into products more easily removed, and passing the treated acetonitrile through a series of adsorbent beds to remove the resulting products and other impurities present from the acetonitrile.

The pretreatment step has two embodiments for the reagent used for the conversion of the unsaturated nitriles. The acetonitrile can be pretreated with a strong base, or a polymerisation initiator to selectively convert the unsaturated nitriles.

In one embodiment of the invention, the acetonitrile is pretreated with a strong base such as alkali hydroxides. In the process of this invention, the hydroxide catalyses the condensation of the unsaturated nitriles to aromatic pyrimidines as well as initiating formation of some polymeric species. The use of solid alkali metal hydroxide, which is largely insoluble in acetonitrile, initiates reactions at the solid-liquid interface, facilitating the removal of the product impurities. The solid alkali metal hydroxide can also act as a bulk dehydrating agent. The unexpected advantages of this invention as practised with solid alkali metal hydroxide as pretreatment reagent include remarkably low levels of hydrolysis and decomposition of acetonitrile, as compared with the use of aqueous base solutions, and resultant high product yields.

Suitable strong bases include, but are not restricted to, alkali metal hydroxides, basic aluminas, hydroxide ion-exchanged anion exchange resins, metal oxide catalysts such as chromium oxide/zinc oxide/aluminium oxide, other basic metal oxides, and mixtures thereof. Preferred strong bases are sodium hydroxide and potassium hydroxide, with potassium hydroxide particularly preferred.

The strong base is added to the acetonitrile in a batch or continuous process to provide an amount of base approximately 0.1 to 1000 moles per mole of total unsaturated nitrile, with 0.5 to 100 moles being preferred.

Another embodiment of the invention includes pretreatment with solid polymerisation initiators to selectively convert the unsaturated nitriles to either high polarity compounds or polymer. One advantage that results from the use of polymerisation initiators as pretreatment reagents include rapid reaction rates. Another advantage is the formation of polymeric material from the UV-adsorbing material in the acetonitrile, such polymeric material being largely insoluble in the acetonitrile, thus making their removal by decantation, centrifugation, or filtration relatively easy and making the efficiency of adsorbent treatment of the acetonitrile relatively efficient when compared with procedures known in the art.

Suitable polymerisation initiators are any which rapidly and selectively polymerise olefinic compounds and which are relatively unreactive with acetonitrile and other aliphatic nitriles. Examples of polymerisation initiators include, but are not restricted to, azo-nitriles such as AIBN (azobis(isobutyronitrile)); alkyl, aryl, and acyl peroxides such as benzoyl peroxide; hydroperoxides and ketone peroxides; peresters and peroxycarbonates; alkali metal and alkaline earth persulfates, such as potassium persulfate; organic sulfonyl azides, and mixtures thereof. The preferred polymerisation initiator is potassium persulfate.

The polymerisation initiator is added to acetonitrile in a batch or continuous process to provide an amount of polymerisation initiator approximately 0.1 to 5.0 moles per mole of C=C or C=O containing material, with 1.0 to 3.0 moles being particularly preferred.

Appropriate temperatures for any of the pretreatment steps are between about 0°C and about 80°C, with temperatures between 0°C and 70°C being preferred, and temperatures between 0°C and 30°C being particularly preferred. The choice of temperature is dependent on the reagent. When a base is used as the reagent, temperatures near ambient temperatures are preferred. Many polymerisation initiators react by prior thermal decomposition to generate active initiator species, and a higher temperature is chosen to provide an adequate decomposition rate.

In a preferred mode, the pretreatment is carried out in the liquid phase in a stirred tank reactor or flow reactor. Reaction times are chosen to provide complete conversion of unsaturated impurities to adsorbable products. The reaction time is from 1 to 10 hours.

The pretreated acetonitrile is then either decanted or filtered as necessary to remove precipitate or delivered directly to the first of the adsorbent beds. Any other conventional means known in the art may be utilised to remove the precipitate.

After the pretreatment step is performed, the acetonitrile is passed through a series of adsorbent beds. The beds are specifically designed to remove the resulting products from the conversion of the unsaturated nitriles and the other impurities present after the pretreatment step. For example, activated charcoal will remove polymer and aromatics. Aluminas, silica, molecular sieves, and alumino-silicates will trap polar organic compounds such as amides and alcohols, as well as functioning as dehydrating agents. The adsorbents can either be regenerated or disposed of in accordance with the application.

Adsorption materials suitable for this invention are activated carbon, large pore zeolites, alumina, silica, alumino-silicates, and similar materials known to the art for selective adsorption of polar materials such as macroporous polymeric resins and the like, and molecular sieves known to the art for selective removal of water from organic materials such as 3A or 4A molecular sieves. The materials can be layered, mixed in the adsorbent beds, or used in pure form in separate adsorbent beds in any order which results in acetonitrile of required purity which is acetonitrile having a UV cutoff of less than 190nm and containing less than 1ppm of water.

The adsorption step can be carried out preferably as a continuous fixed bed process. The adsorbent beds can be operated at ambient temperature or at elevated temperature as required, and with either upward or downward flow. Regeneration of the adsorbent materials will be carried out by known methods such as treatment with streams of dry inert gas such as nitrogen at elevated temperature. In a preferred mode, the adsorbent beds are regenerated by recycling the acetonitrile through the bed and elevating the temperature.

Final filtering of the processed acetonitrile using standard microfiltration technology removes fine particles acquired from the adsorbent materials to yield product meeting the most stringent purity standards.

The process of this invention increases efficiency, energy utilisation, and the overall yield when compared with distillative methods, which require elevated temperatures and long processing times, and which provide relatively low yields of purified product. The high selectivity of adsorbent materials for removal of impurities makes the overall yield of acetonitrile unexpectedly high.

The following examples illustrate the process in accordance with this invention for acetonitrile purification comprising a pretreatment step and an adsorption step. The following examples demonstrate, but do not limit the instant invention.

### EXAMPLE 1

### Purification of Acetonitrile via Base Digestion/Adsorption Process

An 8.0 litre batch reactor vessel equipped with stirrer and nitrogen inlet and outlet was filled with 4.0 litres of product acetonitrile containing 0.05% water and ppm levels of allyl alcohol, acrylonitrile, crotonitrile, and acetamide. To this was added 4.0 grams of dry potassium hydroxide powder (100-200 mesh). The resulting mixture was then stirred at 25°C under nitrogen for 5 hours. The solution was then filtered and pumped through a fixed bed containing 100.0cc of activated carbon, followed by a second bed containing 100cc of Zeolite Type 13X available from UOP Inc. of Des Plaines, Illinois, at a rate of 0.5 litres/hour, corresponding to an LHSV of 2.5 (v/v/hr). Final filtration through a microfiltration membrane removed fine particulate matter picked up from the adsorbent beds. The resulting acetonitrile was analysed by gas chromatography. Karl Fisher Titration showed the acetonitrile to contain less than 30ppm of water. Gas chromatography showed the acetonitrile to contain <0.1 ppm each of allyl alcohol, acrylonitrile, crotonitrile, and acetamide and has a UV cutoff of <190nm. UV Gradient analysis yielded no peaks greater than 5mAU at 210nm.

### EXAMPLE 2

### Purification of Acetonitrile via Selective Polymerization/Adsorption Process

Solid potassium persulfate (5mmol) is added to a sample of acetonitrile (51.3g) containing 5250ppm water, 535ppm acrylonitrile, 650ppm crotonitrile (cis-trans mixture), and 570ppm allyl alcohol. The solution is stirred at room temperature and monitored by gas chromatography. Gas chromatography shows substantial conversion of acrylonitrile, crotonitrile, and allyl alcohol. After filtration of the product solution, UV spectra of the starting material and product supernatant liquid shows dramatic decreases in UV absorption in the range of 190-300nm after potassium persulfate treatment.

The persulfate-treated acetonitrile is then passed over an adsorbent bed of activated carbon, followed by an adsorbent bed of Zeolite Type 13X available from UOP Inc. of Des Plaines, Illinois, followed by an adsorbent bed of 3A molecular sieves. The product acetonitrile contains less than 30ppm of water and has a UV cutoff below 190nm.

## Claims

1. A process for purifying acetonitrile containing impurities comprising water and unsaturated nitriles and other materials with C=C and C=O functional groups at least one of which is selected from acrylonitrile, crotonitrile, acetamide, and allyl alcohol which process comprises the steps of:
(a) pretreating the acetonitrile by adding a solid reagent to selectively convert the impurities into products which are capable of being more easily removed from the acetonitrile by adsorption, the solid reagent being either a strong base or a polymerisation initiator,
(b) passing the treated acetonitrile containing the resulting products and water through a series of adsorbent beds to remove the products and water from the acetonitrile, and
(c) recovering the purified acetonitrile.

2. The process for purifying acetonitrile of claim 1, wherein the solid reagent is a strong base.

3. The process for purifying acetonitrile of claim 2, wherein the strong base is selected from the group consisting of alkali metal hydroxides, basic aluminas, hydroxide ion-exchanged anion exchange resins, metal oxide catalysts, basic metal oxides, and combinations thereof.

4. The process for purifying acetonitrile of claim 2, wherein the strong base is selected from the group consisting of sodium hydroxide, potassium hydroxide, and combinations thereof.

5. The process for purifying acetonitrile of claim 2, wherein the strong base is potassium hydroxide.

6. The process according to any one of claims 2 to 5, wherein the strong base is added to the acetonitrile to provide an amount of base in the range of 0.1 to 1000 moles per mole of total unsaturated nitrile.

7. The process for purifying acetonitrile of claim 6, wherein the strong base is added to the acetonitrile to provide an amount of base in the range of 0.5 to 100 moles per mole of total unsaturated nitrile.

8. The process for purifying acetonitrile of claim 1, wherein the solid reagent is a polymerisation initiator.

9. The process for purifying acetonitrile of claim 8, wherein the polymerisation initiator is selected from the group consisting of azo-nitriles, alkyl peroxides, aryl peroxides, acyl peroxides, hydroperoxides, ketone peroxides, peresters, peroxycarbonates, alkali metal persulfates, alkaline earth persulfates, organic sulfonyl azides, and combinations thereof.

10. The process for purifying acetonitrile of claim 8, wherein the polymerisation initiator is potassium persulfate.

11. The process according to any one of claims 8 to 10, wherein the polymerisation initiator is added to the acetonitrile to provide an amount of initiator in the range of 0.1 to 5.0 moles per mole of C=C or C=O containing material.

12. The process for purifying acetonitrile of claim 11 wherein the polymerisation initiator is added to the acetonitrile to provide an amount of initiator in the range of 1.0 to 3.0 moles per mole of C=C or C=O containing material.

13. The process according to any one of the preceding claims, wherein the adsorbents are selected from the group consisting of activated carbon, large pore zeolites, alumina, silica, alumino-silicates, macroporous polymeric resins, and molecular sieves.

14. The process according to any one of the preceding claims, wherein the temperature for the pretreatment step is between 0°C and 80°C.

15. The process for purifying acetonitrile of claim 14 wherein the temperature for the pretreatment step is between 0°C and 70°C.

16. The process for purifying acetonitrile of claim 15 wherein the temperature for the pretreatment step is between 0°C and 30°C.

17. The process according to any one of the preceding claims, wherein the adsorption step is carried out as a continuous fixed bed process.

## Patentansprüche

1. Verfahren zur Reinigung von Acetonitril, das Verunreinigungen, umfassend Wasser und ungesättigte Nitrile sowie andere Materialien mit funktionellen Gruppen C=C und C=O enthält, wobei mindestens eine Verbindung davon ausgewählt ist aus Acrylnitril, Crotonitril, Acetamid und Allylalkohol, wobei das Verfahren die Schritte umfaßt:
(a) Vorbehandeln des Acetonitrils durch Zugabe eines festen Reagenz zur selektiven Umwandlung der Verunreinigungen zu Produkten, die durch Adsorption leichter aus dem Acetonitril entfernt werden können, wobei das feste Reagenz entweder eine starke Base oder ein Polymerisationsstarter ist,
(b) Leiten des behandelten Acetonitrils, das die erhaltenen Produkte und Wasser enthält, durch eine Reihe von Adsorptionsmittel-Betten zur Entfernung der Produkte und Wasser aus dem Acetonitril, und
(c) Gewinnen des gereinigten Acetonitrils.

2. Verfahren zur Reinigung von Acetonitril nach Anspruch 1, wobei das feste Reagenz eine starke Base ist.

3. Verfahren zur Reinigung von Acetonitril nach Anspruch 2, wobei die starke Base ausgewählt ist aus der Gruppe, bestehend aus Alkalimetallhydroxiden, basischen Aluminiumoxiden, Hydroxidionen-ausgetauschten Anionenaustauschharzen, Metalloxidkatalysatoren, basischen Metalloxiden und Kombinationen davon.

4. Verfahren zur Reinigung von Acetonitril nach Anspruch 2, wobei die starke Base ausgewählt ist aus der Gruppe, bestehend aus Natriumhydroxid, Kaliumhydroxid und Kombinationen davon.

5. Verfahren zur Reinigung von Acetonitril nach Anspruch 2, wobei die starke Base Kaliumhydroxid ist.

6. Verfahren nach einem der Ansprüche 2 bis 5, wobei die starke Base zu dem Acetonitril unter Bereitstellung einer Basenmenge im Bereich von 0,1 bis 1000 Mol pro Mol des gesamten, ungesättigten Nitrils gegeben wird.

7. Verfahren zur Reinigung von Acetonitril nach Anspruch 6, wobei die starke Base zu dem Acetonitril unter Bereitstellung einer Basenmenge im Bereich von 0,5 bis 100 Mol pro Mol des gesamten, ungesättigten Nitrils gegeben wird.

8. Verfahren zur Reinigung von Acetonitril nach Anspruch 1, wobei das feste Reagenz ein Polymerisationsstarter ist.

9. Verfahren zur Reinigung von Acetonitril nach Anspruch 8, wobei der Polymerisationsstarter ausgewählt ist aus der Gruppe, bestehend aus Azonitrilen, Alkylperoxiden, Arylperoxiden, Acylperoxiden, Hydroperoxiden, Ketonperoxiden, Perestern, Peroxycarbonaten, Alkalimetallpersulfaten, Erdalkalimetallpersulfaten, organischen Sulfonylaziden und Kombinationen davon.

10. Verfahren zur Reinigung von Acetonitril nach Anspruch 8, wobei der Polymerisationsstarter Kaliumpersulfat ist.

11. Verfahren nach einem der Ansprüche 8 bis 10, wobei der Polymerisationsstarter zu dem Acetonitril unter Bereitstellung einer Startermenge im Bereich von 0,1 bis 5,0 Mol pro Mol des C=C oder C=O enthaltenden Materials gegeben wird.

12. Verfahren zur Reinigung von Acetonitril nach Anspruch 11, wobei der Polymerisationsstarter zu dem Acetonitril unter Bereitstellung einer Startermenge im Bereich von 1,0 bis 3,0 Mol pro Mol des C=C oder C=O enthaltenden Materials zugegeben wird.

13. Verfahren nach einem der vorangehenden Ansprüche, wobei die Adsorptionsmittel ausgewählt sind aus der Gruppe, bestehend aus Aktivkohle, großporigen Zeolithen, Aluminiumoxid, Siliciumdioxid, Aluminosilicaten, makroporösen Polymerharzen und Molekularsieben.

14. Verfahren nach einem der vorangehenden Ansprüche, wobei die Temperatur für den Vorbehandlungsschritt zwischen 0°C und 80°C liegt.

15. Verfahren zur Reinigung von Acetonitril nach Anspruch 14, wobei die Temperatur für den Vorbehandlungsschritt zwischen 0°C und 70°C liegt.

16. Verfahren zur Reinigung von Acetonitril nach Anspruch 15, wobei die Temperatur für den Vorbehandlungsschritt zwischen 0°C ünd 30°C liegt.

17. Verfahren zur Reinigung nach einem der vorangehenden Ansprüche, wobei der Adsorptionsschritt als kontinuierliches Festbettverfahren ausgeführt wird.

## Revendications

1. Un procédé de purification d'acétonitrile contenant des impuretés comportant de l'eau et des nitriles non saturés et d'autres matières avec des groupes fonctionnels C=C et C=O dont un au minimum est sélectionné à partir de l'acrylonitrile, du crotonitrile, de l'acétamide et de l'alcool allylique, ledit procédé comportant les étapes suivantes :
(a) prétraitement de l'acétonitrile en y ajoutant un réactif solide pour la conversion sélective des impuretés en produits qui sont capables d'être extraits plus aisément de l'acétonitrile par adsorption, le réactif solide étant soit une base forte, soit une amorce de polymérisation ;
(b) passage de l'acétonitrile traité contenant les produits résultants et l'eau à travers une série de lits adsorbants pour extraire de l'acétonitrile les produits et l'eau ; et
(c) récupération de l'acétonitrile purifié.

2. Le procédé de purification de l'acétonitrile de la revendication 1, selon lequel le réactif solide est une base forte.

3. Le procédé de purification de l'acétonitrile de la revendication 2, selon lequel la base forte est sélectionnée parmi le groupe comportant les hydroxydes de métaux alcalins, les alumines basiques, les résines échangeuses d'anions avec ions hydroxydes échangés, les catalyseurs d'oxydes métalliques, les oxydes métalliques basiques et des combinaisons de ceux-ci.

4. Le procédé de purification de l'acétonitrile de la revendication 2, selon lequel la base forte est sélectionnée parmi le groupe comportant l'hydroxyde de sodium, l'hydroxyde de potassium et des combinaisons de ceux-ci.

5. Le procédé de purification de l'acétonitrile de la revendication 2, selon lequel la base forte est l'hydroxyde de potassium.

6. Le procédé selon l'une quelconque des revendications 2 à 5, selon lequel la base forte est ajoutée à l'acétonitrile pour produire une quantité de base dans la plage de 0,1 à 1000 moles par mole de nitrile non saturé total.

7. Le procédé de purification de l'acétonitrile de la revendication 6, selon lequel la base forte est ajoutée à l'acétonitrile pour produire une quantité de base comprise dans la plage de 0,5 à 100 moles par mole de nitrile non saturé total.

8. Le procédé de purification de l'acétonitrile de la revendication 1, selon lequel le réactif solide est une amorce de polymérisation.

9. Le procédé de purification de l'acétonitrile de la revendication 8, selon lequel l'amorce de polymérisation est sélectionnée à partir du groupe comportant des azonitriles, peroxydes alkyles, peroxydes aryles, peroxydes acyles, hydroperoxydes, peroxydes cétoniques, peresters, peroxycarbonates, persulfates de métaux alcalins, persulfates de terres alcalines, azides sulfuryles organiques et des combinaisons de ceux-ci.

10. Le procédé de purification de l'acétonitrile de la revendication 8, selon lequel l'amorce de polymérisation est le persulfate de potassium.

11. Le procédé de purification selon l'une quelconque des revendications 8 à 10, selon lequel l'amorce de polymérisation est ajoutée à l'acétonitrile pour produire une quantité d'amorce comprise dans la plage de 0,1 à 5,0 moles par mole de matière contenant C=C ou C=O.

12. Le procédé de purification de l'acétonitrile de la revendication 11, selon lequel l'amorce de purification est ajoutée à l'acétonitrile pour produire une quantité d'amorce comprise dans la plage de 0,1 à 3,0 moles par mole de matière contenant C=C ou C=O.

13. Le procédé selon l'une quelconque des revendications précédentes, selon lequel les adsorbants sont sélectionnés à partir du groupe comportant le charbon actif, les zéolites à gros pores, l'alumine, la silice, les alumino-silicates, les résines polymères macroporeuses et les tamis moléculaires.

14. Le procédé selon l'une quelconque des revendications précédentes, selon lequel la température de l'étape de prétraitement est comprise entre 0°C et 80°C.

15. Le procédé de purification de l'acétonitrile de la revendication 14, selon lequel la température de l'étape de prétraitement est comprise entre 0°C et 70°C.

16. Le procédé de purification de l'acétonitrile de la revendication 15, selon lequel la température de l'étape de prétraitement est comprise entre 0°C et 30°C.

17. Le procédé de purification de l'acétonitrile selon l'une quelconque des revendications précédentes, selon lequel l'étape d'adsorption est réalisée sous forme d'un procédé sur lit fixe continu.
